# EUROPÄISCHE PATENTSCHRIFT

(11) **EP 2 250 138 B1**
(45) Veröffentlichungstag und Bekanntmachung des Hinweises auf die Patenterteilung: **27.11.2013**
(21) Anmeldenummer: 09708521.1
(22) Anmeldetag: 04.02.2009
(51) Int. Cl.: C04B 35/111, C04B 35/14, C04B 35/447, C04B 35/486, C04B 35/488, B22F 3/11, B22F 3/115, A61L 27/30

(54) **BIOKOMPATIBLES BAUTEIL UND VERFAHREN ZU DESSEN HERSTELLUNG**
BIOCOMPATIBLE COMPONENT AND METHOD FOR PRODUCING THE SAME
COMPOSANT BIOCOMPATIBLE ET PROCÉDÉ DE FABRICATION

(30) Priorität: 08.02.2008 DE 102008008219
(43) Veröffentlichungstag der Anmeldung: 17.11.2010
(73) Patentinhaber: Fraunhofer-Gesellschaft zur Förderung der angewandten Forschung e.V., 80686 München (DE); EMPA Eidgenössische Materialprüfungs- und Forschungsanstalt, 8600 Dübendorf (CH)
(72) Erfinder: ROTA, Astrid, 89415 Lauingen (DE); IMGRUND, Philipp, 28215 Bremen (DE); BRUININK, Arend, CH-8307 Effretikon (CH)
(74) Vertreter: Eisenführ, Speiser & Partner
(86) Internationale Anmeldenummer: PCT/EP2009/000740
(87) Internationale Veröffentlichungsnummer: WO 2009/098036

(56) Entgegenhaltungen:
- DE-A1- 19 943 103
- US-A1- 2002 190 048
- US-A1- 2005 121 417
- US-A1- 2006 211 802
- ROTA A ET AL: "Micro powder metallurgy for the replicative production of metallic microstructures" MICROSYSTEM TECHNOLOGIES SPRINGER-VERLAG GERMANY, Bd. 8, Nr. 4-5, August 2002 (2002-08), Seiten 323-334, XP002549007 ISSN: 0946-7076
- ROTA A: "Funktionalisierte Mikroteile durch Mikro- Metallpulver- Spritzgiessen [Functional micro parts via micro metal injection molding]" INDUSTRIE-MANAGEMENT, GITO-VERLAG, DE, Bd. 21, Nr. 6, 1. Dezember 2005 (2005-12-01), Seiten 53-56, XP008112568 ISSN: 1434-1980

## Beschreibung

### Technisches Gebiet

Die Erfindung betrifft ein Verfahren zur Herstellung von biokompatiblen Bauteilen oder Bauteilen mit biokompatiblen Oberflächenbereichen, insbesondere implantierbaren Bauteilen und Teilen für klinische und Zellkultur-Anwendungen. Die Erfindung betrifft ferner biokompatible Bauteile, die mittels dieses Verfahrens herstellbar sind.

### Stand der Technik

Implantierbare künstliche Materialien und Vorrichtungen sowie Bauteile für klinische und Zellkultur Anwendungen spielen heute im Gesundheitswesen eine wichtige Rolle und besitzen ein wachsendes Potential zur Verbesserung der Pflege- und der Lebensqualität von Menschen und Tieren.

Ein Hauptproblem bei der Entwicklung von Implantaten ist die Biokompatibilität des Implantats mit dem Körper, insbesondere, wenn Gewebe in Kontakt mit dem Implantat kommt.

Ein Verlust der Implantatfunktion kann insbesondere dann erfolgen, wenn die Funktion des Implantats nicht unterstützt wird; wenn also die Anhaftung von Zellen auf dem Implantat, die Vermehrung der Zellen (Proliferation, Differenzierung) und/oder die Bedeckung der Implantate (d.h. das Überwachsen der Implantatoberfläche) nicht gewährleistet ist. Ist beispielsweise bei einem künstlichen Hüftgelenk oder Dentalimplantat ein Überwachsen der Oberfläche mit dem gewünschten Zelltyp nicht möglich, so kann das Implantat nicht in den Knochen eingebaut (osteointegriert) werden.

Die Hauptaufgabe bei der Entwicklung neuer Implantate ist also eine Optimierung der Oberfläche des Implantats in Bezug auf ihre Leistungsfähigkeit im lebenden Organismus, so dass die Körperreaktion derart gesteuert wird, dass das Implantat angenommen wird (und keine Abstossungsreaktion erfolgt) oder, im Fall von Langzeitimplantaten, sogar integriert wird.

Die Integration eines Implantats basiert auf folgenden Ablaufphasen: 1. Anziehung geeigneter Zellen, 2. Proliferation dieser Zellen, um ein Überwachsen des Implantats durch diese Zellen zu erreichen, 3. Differenzierung dieser Zellen in den jeweils für das Implantat gewünschten Zelltyp.

Die Adhäsion von Zellen auf einer bestimmten Oberfläche sowie deren Ausbreitung, Form, Migration, Proliferation und Differenzierung wird durch die Oberflächenstruktur und die Chemie des Oberflächenmaterials wesentlich beeinflusst (vgl. z.B. Bruinink et al., Adv. Eng. Mat. 2005, 7, 411 ff.). Beispielsweise hat eine Strukturierung von knochenbezogenen Implantaten durch Sandstrahlen einen positiven Effekt auf das Implantatverhalten in vivo sowie die Zellentwicklung in vitro. Auch eine Strukturierung der Oberfläche mittels Ätztechniken und Coating-Verfahren führt wie beim Sandstrahlen zu zufällig verteilten Strukturen auf den jeweiligen Oberflächen. Mittels regelmässiger Strukturen können weitere positive Effekte erzielt werden (vgl. Bruinink et al., Adv. Eng. Mat. 2005, 7, 411 ff).

Die US 4,612,160 beschreibt, dass Bauteile mit porösen Oberflächen für medizinische Anwendungen sehr vorteilhaft sind. Es wird daher ein pulvermetallurgisches Verfahren zur Herstellung von Bauteilen, z.B. Implantaten, mit porösen Beschichtungen offenbart, bei dem ein Metallpulver in eine Form, die ein zu beschichtendes Substrat enthält, eingebracht wird und bei dem anschliessend Metallpulver und Substrat in der Form miteinander versintert werden.

Die WO 2006/063354 A1 beschreibt ein mittels eines Spritzgussverfahrens herstellbares Implantat, das aus einem Metallpulver und einem organischen Binder hergestellt wird und auf dessen Oberflächen Poren mit einem durchschnittlichen Durchmesser zwischen 50 µm und 300 µm erzeugt werden können. Alternativ kann auch auf einen Binder verzichtet werden und das Bauteil mittels kaltisostatischen Pressens (CIP) mit anschliessendem Vakuumsintern und heissisostatischen Pressens (HIP) hergestellt werden.

Die US 5,336,465 beschreibt ein Verfahren zur Herstellung von Bauteilen mit guter Biokompatibilität, bei dem ein Gemisch aus pulverförmiger Titanlegierung und einem Binder mittels eines Spritzgussverfahrens in eine Spritzform eingebracht wird und anschliessend eine Entbinderung und Sinterung erfolgen. Die dabei verwendete Mischung besteht aus Partikeln mit einer durchschnittlichen Partikelgrösse von 20 µm.

Die US 2005/0121417 beschreibt ein Bauteil mit guter Biokompatibilität, das eine Mikrostruktur und eine Nanostruktur aufweist.

### Beschreibung

Der vorliegenden Erfindung liegt die Aufgabe zugrunde, ein Verfahren bereitzustellen, mit dem verbesserte Bauteile für biomedizinische Geräte oder implantierbare Materialien bereitgestellt werden können. Insbesondere sollen diese Bauteile gewährleisten, dass eine verbesserte Adhäsion, Ausbreitung und/oder Proliferation der biologischen Zellen auf dem Bauteil erfolgen kann.

Diese Aufgabe wird durch das Verfahren gemäss Anspruch 1 und das Bauteil gemäss Anspruch 11 gelöst. Unteransprüche geben vorteilhafte Weiterbildungen an.

Das erfindungsgemässe Verfahren beruht auf der Erkenntnis, dass eine bessere Integration von Implantaten erreicht werden kann, indem auf der Oberfläche des Implantats keine Nischen für Bakterien, die für Makrophagen (Fresszellen) nicht zugänglich sind, zur Verfügung stehen.

Derart ausgebildete Bauteile können erhalten werden, indem ein Gemisch aus einem pulverförmigen biokompatiblen Material und einem Binder mittels eines Spritzgussverfahrens in eine Spritzform eingebracht oder mittels eines Spritzgussverfahrens auf eine hierdurch zu beschichtende Oberfläche aufgebracht wird und bei dem das so erhaltene Grünteil anschliessend entbindert und gesintert wird. Um eine Ausbildung von Poren ungeeigneter Grösse zu verhindern, wird hierfür als biokompatibles Material eine Mischung, die aus Mikropartikeln und bis zu 50 Gew.-% Nanopartikeln besteht oder die Mikropartikel und bis zu 50 Gew.-% Nanopartikel enthält, eingesetzt. Unter Mikropartikeln werden hierbei Partikel mit einer Partikelgrösse zwischen 700 nm und 50 µm verstanden; Nanopartikel sind Partikel mit einer Partikelgrösse kleiner 700 nm. Die Partikelgrösse wird erfindungsgemäss generell bestimmt mittels Laserbeugung nach ISO-13320-1:1999-1 für Mikropartikel und mittels einer Scheibenzentrifuge für Nanopartikel.

Erfindungsgemäss wird unter einem Bauteil, das zumindest teilweise biokompatible Oberflächenbereiche aufweist, auch ein Bauteil, das vollständig aus einem biokompatiblen Material besteht und einem Bauteil, dessen gesamte Oberfläche aus einem biokompatiblen Material besteht, verstanden (nachfolgend werden derartige Bauteile als biokompatible Bauteile "bezeichnet). Als biokompatible Bauteile kommen insbesondere künstliche implantierbare Materialien und Vorrichtungen sowie Bauteile und Vorrichtungen für klinische und Zellkultur-Anwendungen in Betracht. Hierzu zählen beispielsweise medizinische Implantate (z.B. künstliche Gelenke) und Sensoren sowie Geräte und Bauteile für den Umgang und die Modifikation von biologischen Systemen (z.B. Zellkultursubstrate, Bioreaktoren, Tissue-Engineering-Gewebe und bioelektronische Schnittstellen).

Erfindungsgemäss wird unter Biokompatibilität das Ausbleiben einer unerwünschten Wirkung auf die biologische Umgebung (also das Ausbleiben einer Wirkung, die einen negativen Einfluss auf die Überlebensfähigkeit des Gewebes, das mit dem biokompatiblen Material Kontakt hat - z. B. ist Toxizität zu nennen) verstanden. Biokompatibilität kann zusätzlich auch über das Auftreten eines für das Implantat notwendigen gewünschten Effektes (z.B Integration im Knochen bei einem Hüftgelenk-Implantat) definiert werden.

Mit dem erfindungsgemässen Verfahren werden biokompatible Bauteile erhalten, die nur einen sehr geringen Anteil von Poren mit einem Durchmesser, der grösser als 0,1 µm und kleiner als 1 µm ist, aufweisen. Der Anteil an Poren mit einem Durchmesser kleiner 1 µm und grösser 0,1 µm an der Gesamtzahl der gemessenen Poren sollte mindestens 85 %, bevorzugt 95% betragen. Besonders bevorzugt sind keine Poren mit einem Durchmesser grösser 1 µm messbar. Der Porendurchmesser wird hierbei erfindungsgemäss mittels optischer Auswertung eines metallographischen Schliffs ermittelt.

Das erfindungsgemässe Verfahren hat daher den Vorteil, dass Oberflächen hergestellt werden können, die für Anhaftung, Vermehrung, Differenzierung und Bedeckung der Oberflächen durch Zellen besser geeignet sind als die nach dem Stand der Technik. Statt einer stochastischen Verteilung der Oberflächenstrukturen und deren Grössen können mittels des vorliegenden Verfahrens definierte und eine auf die Zellen abgestimmte Strukturierung hergestellt werden. Zudem können sich im Fall von Implantaten aufgrund fehlender Nischen für Bakterien, die für Abwehrzellen nicht zugänglich sind, keine chronischen Entzündungen entwickeln. Erfahrungsgemäss treten solche Entzündungen in der Hälfte aller Fälle etwa ein Jahr nach Implantation auf, also unabhängig von der Implantations-Operation.

Trotzdem werden mit dem erfindungsgemässen Verfahren Oberflächen zur Verfügung gestellt, die im Nanometerbereich strukturiert sind, was für eine optimale Adhäsion, Zellverhalten und/oder Funktionalität notwendig ist. Optimal bedeutet bei Adhäsion eine Anhaftung von Zellen der Osteoblast Linie welche hoch genug ist, um eine gute Ausbreitung auf dem Implantat zu unterstützen aber nicht so hoch, dass es eine Zellmigration gehemmt wird. Optimal beim Zellverhalten bedeutet, dass Migration und Zellteilung in dem Mass unterstützt wird, dass die Zellen der Osteoblast Linie die Oberflächen einigermassen selektiv abdecken. Optimal in Sinne von Funktionalität bedeutet, dass die Zellen in einen Zustand versetzt werden, bei dem Knochenmaterial an der Implantatoberfläche abgelagert wird.

Ausserdem hat das erfindungsgemässe Verfahren auch den Vorteil, dass - aufgrund der eingesetzten Nanopulver - eine Oberflächenstruktur im Nanometerbereich erzeugt wird. Eine Nanostrukturierung kann nach dem Stand der Technik allenfalls durch einen zusätzlichen Bioaktivierungsschritt hergestellt werden; d.h. es ist ein zusätzlicher Verfahrensschritt nötig, der die Teilefertigung wesentlich verteuert. Eine Bioaktivierung ist nach dem Stand der Technik beispielsweise nötig, wenn zur Herstellung von biokompatiblen Bauteilen klassische Mikrofertigungsverfahren für Metalle und Keramiken (z.B. Mikrofräsen, Mikro-Erudieren oder Lasertechniken zur Herstellung geeigneter Oberflächen) eingesetzt werden. Meist erlauben diese Mikrofertigungs-Verfahren auch keine Strukturierung bis in den Nanobereich und sind zudem unflexibel bezüglich Serienfertigung.

Der neue Fertigungsansatz erlaubt es, frei definierbare Materialoberflächen zu erzeugen, die dann Eigenschaften aufweisen, die für die Funktionalität und das Verhalten der jeweils relevanten biologischen Zellen optimal sind.

Bevorzugt beträgt der Anteil der Nanopartikel im biokompatiblen Material 5 bis 50 Gew.%, besonders bevorzugt 15 bis 35 Gew.-%. Bei Gehalten kleiner 5 Gew.-% ist es schwierig, die gewünschte Nanostrukturierung des Bauteils zu realisieren; Gehalte grösser 50 Gew.-% sind aus Kostengründen ungünstig. Im Bereich zwischen 15 und 35 Gew.-% fallen keine zu hohen Kosten bei gleichzeitig guter Strukturausbildung an. Weiterhin bevorzugt werden Nanopartikel mit einer Grösse von 10 bis 500 nm eingesetzt. Diese sind im Hinblick auf die Zellhaftung besonders geeignet.

Das erfindungsgemässe Verfahren wird derart durchgeführt, dass aus dem pulverförmigen biokompatiblen Material und dem Binder eine Mischung hergestellt wird. Bevorzugt erfolgt dies so, dass die Partikel des pulverförmigen biokompatiblen Materials vollständig mit dem Binder umhüllt werden (beispielsweise durch Mischen bei erhöhter Temperatur). Bezüglich näherer Details wird hier auf die DE 199 35 276 A1 verwiesen. Dies hat den Vorteil, dass die Luftempfindlichkeit der Pulver stark herabgesetzt wird. Dementsprechend erfolgt der primäre Mischungsprozess bevorzugt in einer Schutzatmosphäre aus Schutzgas und/oder Luftausschluss und/oder Vakuum. Um ein Verkleben der Binderbestandteile und der Bestandteile des biokompatiblen Pulvers zu verhindern, kann ein Gleitmittel zugefügt werden. Um eine möglichst vollständige Benetzung der Pulverpartikel zu erhalten, können Benetzungsmittel (surfactants) zugesetzt werden. Weiterhin kann die Vermischung in einem Kneter erfolgen, um eine ausreichend homogene Vermischung zu gewährleisten, ohne dass die Bestandteile verklumpen.

Anschliessend erfolgt der Pulverspritzguss des Bauteils in einer Spritzgussmaschine bzw. das Aufbringen der Oberflächenbeschichtung mittels eines Spritzgussverfahrens. Dieser Verfahrensschritt erfolgt üblicherweise mittels eines Pulverspritzgussverfahrens (PIM), kann aber z.B. auch mittels Prägen von Pulver-Binder-Mischungen oder Niederdruckspritzguss erfolgen.

Schliesslich erfolgt die Entbinderung und Sinterung des so hergestellten Bauteils. Zur Entbinderung kann beispielsweise zunächst eine Teilentbinderung vorgenommen werden. Dies erfolgt insbesondere durch thermisches Austreiben oder chemische Extraktion (vorzugsweise unter Schutzgasatmosphäre, Wasserstoffatmosphäre oder im Vakuum).

Die Entbinderung und das Sintern kann auch in einem Prozessschritt zusammengefasst werden. Das Sintern erfolgt derart, dass die einzelnen Metallpulverteile metallurgische Kontakte in Form einer Schweissdiffusion miteinander erhalten und kann beispielsweise mittels Wärmestrahlungssintern erfolgen.

Bevorzugt erfolgt das Sintern mittels Mikrowellensintern und/oder Sparkplasmasintern. Diese beiden Verfahren haben den Vorteil, dass eine sehr schnelle Erhitzung und Versinterung der Pulverpartikel ermöglicht wird. Damit kann eine sehr feine Korngrösse und Strukturierung der Oberfläche im Bereich kleiner 700 nm genauer und besser eingestellt werden. Das Verfahren wird dabei bevorzugt so durchgeführt, dass möglichst nur ein Zusammenwachsen der Pulverbestandteile in dem Mass erfolgt, wie es für die mechanische Stabilität des erhaltenen Bauteils nötig ist mit der Massgabe dass dabei die vorstehenden Vorgaben bezüglich der Porosität und Oberflächenstrukturierung eingehalten werden.

Bevorzugt wird bei dem Verfahren ein Gemisch aus biokompatiblem Material und Binder eingesetzt, bei dem maximal 40 Gew.-% Binder, bevorzugt 4 bis 30 Gew.-% Binder enthalten sind.

Als Binderbestandteile werden insbesondere thermoplastische und/oder duroplastische Polymere, thermogelierende Substanzen, Wachse oder oberflächenaktive Substanzen oder daraus erhaltene Mischungen eingesetzt. Bevorzugt werden die Binderbestandteile derart ausgewählt, dass die Entbinderung unter möglichst milden Bedingungen erfolgen kann. Insbesondere sollten diese Bedingungen so gewählt werden, dass im fertigen Bauteil eine möglichst ausgeprägte Nanostrukturierung der Oberfläche erhalten wird mit der Massgabe dass auch hier die vorstehenden Vorgaben bezüglich der Porosität eingehalten werden.

Als biokompatibles Material wird insbesondere ein Metall, eine Keramik oder ein Gemisch aus Metall und Keramik eingesetzt. Die biokompatiblen Materialien müssen dabei für den angewendeten Fertigungsprozess geeignet sein, d.h. sinterfähig sein. Bevorzugt werden die Materialien so ausgewählt, dass der Sinterprozess in möglichst kurzer Zeit und unter möglichst milden Bedingungen erfolgen kann. Besonders geeignet sind daher biokompatible metallische Materialien, insbesondere biokompatible Metalle, die ausgewählt sind aus der Gruppe, bestehend aus Edelstahl, Titan, Titanlegierungen und Legierungen auf Chrom-Cobalt-Basis (d.h. Legierungen, die Chrom und Cobalt enthalten oder hieraus bestehen). Weiterhin geeignet sind Keramiken wie ZrO₂, Al₂O₃, SiO₂ und Calciumphosphate.

Das erfindungsgemässe Verfahren wird weiterhin derart durchgeführt, dass neben der Nanostruktur möglichst auch eine Mikrostruktur erzeugt wird.

Unter einer Nanostruktur wird erfindungsgemäss verstanden, dass eine vollständige homogene Strukturierung der Oberfläche mit Strukturelementen vorlegt, die in keiner Raumrichtung eine Ausdehnung grösser als 0,35 µm besitzen. Bevorzugt haben diese Strukturelemente auch in keiner Raumrichtung eine Ausdehnung grösser als 0,1 µm. Von derartigen Strukturelementen mit einem Durchmesser von 100 nm und darunter ist bekannt, dass sie einen positiven Effekt auf Osteoblasten haben ( vgl. T. J. Webster et al. in "Enhanced functions of osteoblasts on nanophase ceramics", Biomaterials 21 (2000), 1803-1810; T. J. Websterand et al. in "Increased osteoblast adhesion on nanophase metals: Ti, Ti6Al4V, and CoCrMo", Biomaterials 25 (2004), 4731-4739). Die Bestimmung der Oberflächengeometrie erfolgt hierbei mittels optischer Auswertung einer berührungslosen 3D-Topographieanalyse.

Die Oberflächenstruktur wird dabei in der Regel nicht durch die Form und Ausdehnung der eingesetzten Pulver bestimmt sondern durch die Korngrösse des entstandenen Gefüges.

Die Nanostruktur kann erfindungsgemäss auch über die Rauheit der biokompatiblen Oberflächenbereiche beschrieben werden. Diese weist bevorzugt einen arithmetischen Mittenrauhwert Ra kleiner 200 nm und eine mittlere Rauhtiefe Rz < 2,5 µm auf. Die Bestimmung dieser Kennwerte erfolgt erfindungsgemäss mittels berührungsloser Linienprofilometrie. Die Kennwerte werden dabei gemäss DIN 4762, DIN 4287/2 und 4288 definiert beziehungsweise ermittelt.

Unter einer Mikrostruktur wird erfindungsgemäss verstanden, dass auf der Oberfläche des biokompatiblen Bauteils nach aussen gewölbte Strukturen (nachfolgend Erhebungen genannt) und/oder diesen Erhebungen entsprechende inverse Strukturen (also Vertiefungen) vorliegen. Diese Erhebungen und/oder Vertiefungen können z.B. nach Art einer Hemisphäre, einer Säule, einer Pyramide oder eines Kegels aber auch nach Art eines Stegs bzw. einer Rille oder den entsprechenden inversen Strukturen vorliegen. Es können dabei auch verschiedenartige Erhebungen und/oder Vertiefungen nebeneinander vorliegen. Die Erhebungen und/oder Vertiefungen können einen mittleren Durchmesser von 5 bis 200 µm, bevorzugt 10 bis 50 µm, aufweisen. Der maximale Durchmesser der Erhebung sollte dabei 350 µm nicht überschreiten. Der Durchmesser wird hierbei mittels optischer Auswertung einer berührungslosen 3D-Topographieanalyse mit einer Mindestauflösung von 1 µm ermittelt. Die einzelnen Mikrostrukturelemente bleiben dabei aber nicht auf Hemisphären, Säulen, Pyramiden, Kegeln und Stege begrenzt; grundsätzlich sind jegliche geometrischen Formen denkbar, die z.B. von einer Kugel, einem Ei, einem Rotationsellipsoid, aber auch von Würfeln, Kegeln, Stegen und dergleichen abgeleitet sind. Stege können beispielsweise auch ringförmig ausgebildet sein. Die Höhe dieser Erhebungen und/oder Vertiefungen beträgt im Verhältnis zum mittleren Durchmesser etwa 30 bis 100 %, bevorzugt 25 bis 50 %, und wird mittels berührungsloser 3D-Topographieanalyse bestimmt.

Der Abstand der einzelnen Mikrostrukturelemente beträgt (vom Schwerpunkt der einzelnen Kavität aus gemessen) 5 bis 200 µm, bevorzugt 10 bis 50 µm und wird mittels berührungsloser 3D-Topographieanalyse gemessen.

Das Vorliegen einer Mikrostruktur und einer Nanostruktur hat den Vorteil, dass ein noch gezielteres Steuern des Zellverhaltens und der Funktionalität von biologischen Zellen möglich ist.

Die Mikrostrukturierung wird im Regelfall durch Replikation erzeugt. Insbesondere ist es von Vorteil, wenn in die Spritzform Negative (z.B dellenartige Vertiefungen und Rillen) der im Bauteil zu erzeugenden Erhebungen bereits in der Oberfläche integriert sind. Auch die Beschichtung eines Bauteils kommt in Betracht.

Mit den erfindungsgemässen Bauteilen ist es z.B. möglich, Platten oder Plättchen zur Versteifung von Knochen, die ein optimales Auf- und Einwachsen von Knochenzellen fördern, bereitzustellen. Auch komplizierte Implantatgeometrien können aber mit dem erfindungsgemässen Verfahren realisiert werden.

Neben Implantaten kann es auch für biomedizinische Geräte von Vorteil sein, eine mit dem erfindungsgemässen Verfahren hergestellte Oberflächentopdgraphie aufzuweisen. Beispielsweise seien sogenannte Gerüste (scaffolds) zum Einsatz im Tissue-Engineering, um Gewebe oder Organe aus einer Gruppe von Zellen zu generieren, genannt. Ähnliche Beispiele sind Substrate für Zellkulturen, Bioreaktoren, Schnittstellen für elektronische lebensunterstützende Geräte ausserhalb des Körpers. Zum Beispiel kann es von Vorteil sein, eine bestimmte Topographie zu nutzen, um eine erhöhte Mobilltät spezifischer Zellen zu induzieren, so dass diese Zellen sich über das Substrat verteilen können und durch topographische Gegebenheiten zu bestimmten Orten geleitet werden. Weiterhin kann eine Topographie derart ausgebildet sein, dass die Proliferation Differenzierung und/oder Migration der Zellen angeregt werden.

### Beispiele

Ohne Einschränkung der Allgemeinheit wird die Erfindung anhand von Beispielen nachfolgend näher beschrieben:
Titanpulver mit einer mittleren Partikelgrösse von 25 µm wird mit einem Anteil von 20 Gew.% Nanopulver (mittlere Partikelgrösse von 400 nm) versetzt. Diese Mischung wird mit einem Binderanteil von 10 Gew% bei 100 °C unter Luftabschluss in einer Argonatmosphäre vermischt. Der Binder besteht aus 70 % Paraffin, 20% Polyethylen und 10% Carnaubawachs. Diese granulierte Mischung wird nun auf einer Spritzgussmaschine aufgeschmolzen, so dass der Binderanteil flüssig ist, und in eine Form eingespritzt. Als Formen dienen im klassischen Werkzeugbau hergestellte Werkzeugeinsätze, die mittels Mikrofertigungstechniken zusätzlich mit Mikrostrukturen, beispielsweise halbkreisförmigen Kavitäten von 20 µm Durchmesser, versehen wurden. Die Gesamtkavität liegt bei etwa 2.5 cm³. Die so hergestellten Formteile - immer noch bestehend aus Metallpulver und Bindemittel - werden dann in einem Lösungsmittelbad für mehrere Stunden entbindert. Anschliessend erfolgt eine thermische Behandlung in Argon-Atmosphäre, um den Rest des Binders zu entfernen. Der abschliessende Sinterschritt erfolgt in einem konventionellen Sinterofen oder Mikrowellenofen. Im konventionellen Sinterofen beträgt die Heizrate 10 K / min und die Sintertemperatur etwa 1000-1300°C. Die Haltedauer bei Sintertemperatur liegt bei zwei Stunden. Alternativ werden im Mikrowellenofen Heizraten von 50 bis 100 K /min erreicht, die Sinterdauer beträgt eine halbe Stunde. Mit der Sinterung in der Mikrowelle wird bei verkürzten Prozesszeiten eine hohe Verdichtung und geringere Korngrösse angestrebt. Man erhält solide metallische Bauteile, die um die Volumenmenge des zuvor eingebrachten Binders geschrumpft sind. Die Dichte dieser Bauteile wird nach dem Archimedesprinzip bestimmt. Sie beträgt nach der Sinterung etwa 4,35 g/cm³ (97% der theoretischen Dichte).

## Patentansprüche

1. Verfahren zur Herstellung von Bauteilen, die zumindest teilweise biokompatible Oberflächenbereiche aufweisen, bei dem ein Gemisch aus einem pulverförmigen biokompatiblen Material und einem Binder mittels eines Spritzgussverfahrens in eine Spritzform eingebracht oder mittels eines Spritzgussverfahrens auf eine zu beschichtende Oberfläche aufgebracht wird und anschließend eine Entbinderung und Sinterung der erzeugten Bauteile erfolgt, **dadurch gekennzeichnet dass**, als pulverförmiges biokompatibles Material eine Mischung, bestehend aus Partikeln mit einer Partikelgröße zwischen 700 nm und 50 µm (Mikropartikeln) und bis zu 50 Gew.-% Partikeln mit einer Partikelgröße kleiner 700 nm (Nanopartikeln) eingesetzt wird, wobei der Anteil der Nanopartikel in der Mischung zwischen 5 Gew.-% und 50 Gew.-% liegt.

2. Verfahren nach Anspruch 1, **dadurch gekennzeichnet, dass** der Anteil der Nanopartikel in der Mischung zwischen 15 Gew.-% und 35 Gew.-% liegt.

3. Verfahren nach einem der vorhergehenden Ansprüche, **dadurch gekennzeichnet, dass** die Nanopartikel eine Größe von 10 bis 500 nm besitzen.

4. Verfahren nach einem der vorhergehenden Ansprüche, **dadurch gekennzeichnet, dass** das Entbindern und/oder Sintern mittels Mikrowellensintern und/oder Sparkplasmasintern erfolgt.

5. Verfahren nach einem der vorhergehenden Ansprüche, **dadurch gekennzeichnet, dass** das Gemisch aus dem biokompatiblen Material und dem Binder maximal 40 Gew.-% Binder enthält.

6. Verfahren nach einem der vorhergehenden Ansprüche, **dadurch gekennzeichnet, dass** das Gemisch aus dem biokompatiblen Material und dem Binder 4 bis 30 Gew.-% Binder enthält.

7. Verfahren nach einem der vorhergehenden Ansprüche, **dadurch gekennzeichnet, dass** das biokompatible Material ein Metall oder eine Keramik ist.

8. Verfahren nach dem vorhergehenden Anspruch, **dadurch gekennzeichnet, dass** das biokompatible Metall ausgewählt wird aus der Gruppe bestehend aus Edelstahl, Titan, Titanlegierungen und Legierungen auf Chrom-Cobalt-Basis.

9. Verfahren nach einem der Ansprüche 1 bis 7, **dadurch gekennzeichnet, dass** die biokompatible Keramik ausgewählt wird aus der Gruppe bestehend aus ZrO₂, Al₂O₃, SiO₂ und Calciumphosphaten.

10. Verfahren nach einem der vorhergehenden Ansprüche, **dadurch gekennzeichnet, dass** auf den Bauteilen eine Mikrostruktur und eine Nanostruktur erzeugt wird.

11. Verfahren nach einem der vorhergehenden Ansprüche, **dadurch gekennzeichnet, dass** das beim Pulverspritzguss eingesetzte Formgebungswerkzeug und/oder die Spritzform Erhebungen und/oder Vertiefungen zur Erzeugung der Mikrostruktur aufweisen.

12. Bauteil, das zumindest teilweise biokompatible Bereiche sowie eine Vielzahl von Poren aufweist, herstellbar insbesondere nach dem Verfahren nach einem der vorhergehenden Ansprüche, **dadurch gekennzeichnet, dass** das Bauteil eine Mikrostruktur und eine Nanostruktur aufweist und dass der Anteil an Poren mit einem Durchmesser kleiner 1 µm und grösser 0,1 µm an der Gesamtzahl der gemessenen Poren mindestens 85 % beträgt.

13. Bauteil nach Anspruch 12, **dadurch gekennzeichnet, dass** der Anteil an Poren mit einem Durchmesser kleiner 1 µm und grösser 0,1 µm an der Gesamtzahl der gemessenen Poren mindestens 95% beträgt.

14. Bauteil nach Anspruch 12, **dadurch gekennzeichnet, dass** der Anteil an Poren mit einem Durchmesser kleiner 1 µm und grösser 0,1 µm an der Gesamtzahl der gemessenen Poren 100% beträgt.

15. Bauteil nach einem der vorhergehenden Ansprüche, **dadurch gekennzeichnet, dass** die biokompatiblen Oberflächenbereiche einen arithmetischen Mittenrauhwert zwischen Ra = 20 nm und 5 µm sowie eine mittlere Rauhtiefe Rz zwischen 200 nm und 70 µm aufweisen.

## Claims

1. A method for producing components which have at least partially biocompatible surface areas, in which a mixture of a powdered biocompatible material and a binder is introduced into an injection mould by means of an injection moulding method or is applied to a surface to be coated by means of an injection moulding method, and the components produced are then subjected to debinding and sintering, **characterised in that**, as the powdered biocompatible material, a mixture is used which consists of particles having a particle size of between 700 nm and 50 µm (microparticles) and of up to 50 wt.% of particles having a particle size of less than 700 nm (nanoparticles), the proportion of nanoparticles in the mixture being between 5 wt.% and 50 wt.%.

2. The method according to claim 1, **characterised in that** the proportion of nanoparticles in the mixture is between 15 wt.% and 35 wt.%.

3. The method according to one of the preceding claims, **characterised in that** the nanoparticles have a size of 10 to 500 nm.

4. The method according to one of the preceding claims, **characterised in that** the debinding and/or sintering takes place by means of microwave sintering and/or spark plasma sintering.

5. The method according to one of the preceding claims, **characterised in that** the mixture of the biocompatible material and the binder contains no more than 40 wt.% of binder.

6. The method according to one of the preceding claims, **characterised in that** the mixture of the biocompatible material and the binder contains 4 to 30 wt.% of binder.

7. The method according to one of the preceding claims, **characterised in that** the biocompatible material is a metal or a ceramic.

8. The method according to the preceding claim, **characterised in that** the biocompatible metal is selected from the group consisting of stainless steel, titanium, titanium alloys and alloys based on chromium-cobalt.

9. The method according to one of claims 1 to 7, **characterised in that** the biocompatible ceramic is selected from the group consisting of ZrO₂, Al₂O₃, SiO₂ and calcium phosphates.

10. The method according to one of the preceding claims, **characterised in that** a microstructure and a nanostructure are produced on the components.

11. The method according to one of the preceding claims, **characterised in that** the forming tool used in the powder injection moulding and/or the injection mould have protrusions and/or depressions for producing the microstructure.

12. A component having at least partially biocompatible areas and a plurality of pores, which can be produced in particular by the method according to one of the preceding claims, **characterised in that** the component has a microstructure and a nanostructure and that the proportion of pores having a diameter of less than 1 µm and greater than 0.1 µm in the total number of pores measured is at least 85%.

13. The component according to claim 12, **characterised in that** the proportion of pores having a diameter of less than 1 µm and greater than 0.1 µm in the total number of pores measured is at least 95%.

14. The component according to claim 12, **characterised in that** the proportion of pores having a diameter of less than 1 µm and greater than 0.1 µm in the total number of pores measured is 100%.

15. The component according to one of the preceding claims, **characterised in that** the biocompatible surface areas have an arithmetical mean roughness of between Ra = 20 nm and 5 µm and a mean surface roughness Rz of between 200 nm and 70 µm.

## Revendications

1. Procédé de fabrication de composants présentant des zones superficielles au moins partiellement biocompatibles, dans lequel un mélange composé d'un matériau biocompatible pulvérulent et d'un liant est introduit dans un moule à injection au moyen d'un procédé de moulage par injection ou appliqué sur une surface à enduire au moyen d'un procédé de moulage par injection puis un déliantage ou un frittage des composants produits est réalisé, et **caractérisé en ce que** l'on utilise comme matériau biocompatible pulvérulent un mélange composé de particules d'une taille comprise entre 700 nm et 50 µm (microparticules) et contenant jusqu'à 50 % en masse de particules d'une taille inférieure à 700 nm (nanoparticules), la proportion de nanoparticules dans le mélange étant comprise entre 5 % en masse et 50 % en masse.

2. Procédé selon la revendication 1, **caractérisé en ce que** la proportion de nanoparticules dans le mélange est comprise entre 15 % en masse et 35 % en masse.

3. Procédé selon l'une quelconque des revendications précédentes **caractérisé en ce que** les nanoparticules possèdent une taille de 100 à 500 nm.

4. Procédé selon l'une quelconque des revendications précédentes **caractérisé en ce que** le déliantage et/ou le frittage sont effectués à l'aide d'un frittage à micro-ondes et/ou d'un frittage par plasma à étincelles.

5. Procédé selon l'une quelconque des revendications précédentes **caractérisé en ce que** le mélange composé du matériau biocompatible et du liant comprend maximum 40 % en masse de liant.

6. Procédé selon l'une quelconque des revendications précédentes **caractérisé en ce que** le mélange composé du matériau biocompatible et du liant comprend 4 à 30 % en masse de liant.

7. Procédé selon l'une quelconque des revendications précédentes **caractérisé en ce que** le matériau biocompatible est un métal ou une céramique.

8. Procédé selon l'une quelconque des revendications précédentes **caractérisé en ce que** le matériau biocompatible est sélectionné dans un groupe constitué de l'acier inoxydable, du titane, d'alliages de titane et d'alliages à base de chrome-cobalt.

9. Procédé selon l'une des revendications 1 à 7 **caractérisé en ce que** le matériau biocompatible est sélectionné dans un groupe constitué de ZrO₂, d'Al₂O₃, de SiO₂ et de phosphates de calcium.

10. Procédé selon l'une quelconque des revendications précédentes **caractérisé en ce qu'**une microstructure et une nanostructure sont produites sur les composants.

11. Procédé selon l'une quelconque des revendications précédentes **caractérisé en ce qu'**un outil de moulage utilisé pour le moulage par injection de poudre et/ou le moule à injection comprennent des protubérances et/ou des creux en vue de produire la microstructure.

12. Composant présentant des zones au moins partiellement biocompatibles ainsi que de nombreux pores, pouvant être fabriqué notamment suivant le procédé selon l'une quelconque des revendications précédentes, **caractérisé en ce que** le composant présente une microstructure et une nanostructure et que la proportion de pores ayant un diamètre inférieur à 1 µm et supérieur à 0,1 µm par rapport au nombre total de pores mesurés est d'au moins 85 %.

13. Composant selon la revendication 12 **caractérisé en ce que** la proportion de pores ayant un diamètre inférieur à 1 µm et supérieur à 0,1 µm par rapport au nombre total de pores mesurés est d'au moins 95 %.

14. Composant selon la revendication 12 **caractérisé en ce que** la proportion de pores ayant un diamètre inférieur à 1 µm et supérieur à 0,1 µm par rapport au nombre total de pores mesurés est d'au moins 100 %.

15. Composant selon l'une quelconque des revendications précédentes **caractérisé en ce que** les zones superficielles biocompatibles possèdent un écart moyen arithmétique Ra compris entre 20 nm et 5 µm ainsi qu'une profondeur de rugosité moyenne Rz comprise entre 200 nm et 70 µm.
